# EUROPEAN PATENT SPECIFICATION

(11) **EP 3 988 075 B1**
(45) Date of publication and mention of the grant of the patent: **21.08.2024**
(21) Application number: 20844313.5
(22) Date of filing: 19.06.2020
(51) Int. Cl.: A61J 1/20, A61M 39/10

(54) **TO-BE-ADMINISTERED DRUG SOLUTION ADJUSTING TOOL, TO-BE-ADMINISTERED DRUG SOLUTION ADJUSTING KIT, AND METHOD FOR ADJUSTING TO-BE-ADMINISTERED DRUG SOLUTION USING THE SAME**
WERKZEUG ZUR ANPASSUNG EINER ZU VERABREICHENDEN ARZNEIMITTELLÖSUNG, KIT ZUR ANPASSUNG EINER ZU VERABREICHENDEN ARZNEIMITTELLÖSUNG UND VERFAHREN ZUR ANPASSUNG EINER ZU VERABREICHENDEN ARZNEIMITTELLÖSUNG DAMIT
OUTIL DE RÉGLAGE DE SOLUTION MÉDICAMENTEUSE À ADMINISTRER, KIT DE RÉGLAGE DE SOLUTION MÉDICAMENTEUSE À ADMINISTRER, ET PROCÉDÉ DE RÉGLAGE D'UNE SOLUTION MÉDICAMENTEUSE À ADMINISTRER FAISANT APPEL AUXDITS OUTIL ET KIT

(30) Priority: 22.07.2019 JP 2019134495
(43) Date of publication of application: 27.04.2022
(73) Proprietor: TERUMO Kabushiki Kaisha, Tokyo 151-0072 (JP)
(72) Inventor: FUJIWARA Sho, Ashigarakami-gun, Kanagawa 259-0151 (JP); UEDA Tsutomu, Ashigarakami-gun, Kanagawa 259-0151 (JP)
(74) Representative: Casalonga
(86) International application number: PCT/JP2020/024110
(87) International publication number: WO 2021/014848

(56) References cited:
- WO-A1-01/10486
- JP-A- 2001 505 072
- JP-A- 2007 215 775
- JP-A- 2010 279 409
- JP-A- 2014 200 504
- JP-A- S59 150 514
- US-A1- 2005 209 555
- US-A1- 2012 234 757
- US-B1- 6 347 711

## Description

### BACKGROUND

### Technical Field

The present invention relates to an administrable liquid medicine preparation tool, an administrable liquid medicine preparation kit, and an administrable liquid medicine preparation method using the same capable of removing air bubbles in a liquid and having excellent operability.

In particular, the present invention relates to an administrable liquid medicine preparation tool according to the preamble of independent claim 1, such as it is for example known from US 2012/234757 A1 or US 6 347 711 B1.

### Related Art

Conventionally, some injections having low stability in a liquid are provided in a state of being solidified into a powder and the like by a freeze-drying method, a spray-drying method and the like. Such solidified injection is mixed with a medical liquid such as water for injection and physiological saline in a solidified injection container such as an ampule and a vial before being administered to a patient.

When the solidified injection and the medical liquid are mixed, air bubbles might be generated. When the air bubbles are generated, disadvantages such as difficulty in accurately collecting a mixed liquid and remaining of the mixed liquid in the container occur when the mixed liquid is transferred from the container to a syringe and the like at the time of administration. Discharge of the mixed liquid associated with an air vent operation of the syringe after the mixed liquid is transferred to the syringe causes a loss of the mixed liquid.

Furthermore, presence of air bubbles in the mixed liquid (administrable liquid medicine) might cause aggregation and denaturation of ingredients, especially active ingredients of the injection at a gas-liquid interface, and deteriorate medicine efficacy.

JP 2012-228332 (Patent Document 1) discloses a liquid medicine preparation tool capable of eliminating air bubbles generated during a dissolution operation of a powdered preparation.

The liquid medicine preparation tool of Patent Document 1 is provided with a main body including a partition wall, a vial mounting portion including a puncture needle provided on one side of the partition wall, and a collection device mounting port provided on the other side of the partition wall, in which a liquid medicine passage with one end opened to the collection device mounting port and an air passage with one end opened to an outer peripheral surface (external space) of the main body are formed in the puncture needle. According to such liquid medicine preparation tool, a powdered preparation and a solvent are mixed in a state in which the inside of the vial storing the powdered preparation is held at a negative pressure, and in a case where air bubbles are generated in the vial during a dissolution operation, the air passage is communicated with the external space, so that outside air may flow into the vial through the air passage to eliminate the air bubbles in the vial.

[Patent Document 1] JP 2012-228332A

### SUMMARY

However, in the liquid medicine preparation tool as disclosed in JP 2012-228332, air bubbles generated when the powdered preparation and the medical liquid are mixed cannot be sufficiently removed, and various adverse effects due to the air bubbles present in the mixed liquid might not be sufficiently removed.

The present inventors further studied the generation of the air bubbles in the mixed liquid, and found that gas (air bubbles or foam) contained in the medical liquid before being mixed with the powdered preparation also contributes to the generation of the air bubbles in the mixed liquid.

Furthermore, as a result of examining actual use of the liquid medicine preparation tool as disclosed in JP 2012-228332, the present inventors found that it takes time and effort to store the mixed liquid (administrable liquid medicine) in the syringe for administration again after preparing the mixed liquid in the vial, and there is a possibility that new air bubbles are mixed at that time.

Therefore, the present invention provides an administrable liquid medicine preparation tool, an administrable liquid medicine preparation kit, and an administrable liquid medicine preparation method using the same capable of reducing air bubbles in the administrable liquid medicine and facilitating a preparation operation of the administrable liquid medicine.

The above-described object is achieved by an administrable liquid medicine preparation tool according to independent claim 1. The dependent claims relate to advantageous embodiments.

### BRIEF DESCRIPTION OF DRAWINGS

FIG. 1 is a plan view of an administrable liquid medicine preparation tool according to an embodiment of the present invention;
FIG. 2 is a cross-sectional view taken along line A-A in FIG. 1;
FIG. 3 is a plan view of a housing member forming the administrable liquid medicine preparation tool illustrated in FIG. 1;
FIG. 4 is a plan view illustrating a state in which a hydrophilic filtration membrane is attached to the housing member illustrated in FIG. 3;
FIG. 5 is an enlarged view for illustrating an air vent mechanism provided on the administrable liquid medicine preparation tool illustrated in FIG. 1;
FIG. 6 is an illustrative view of an administrable liquid medicine preparation kit according to the embodiment of the present invention;
FIG. 7 is a cross-sectional view for illustrating a step of an administrable liquid medicine preparation method of an embodiment of the present invention using the administrable liquid medicine preparation kit illustrated in FIG. 6;
FIG. 8 is a cross-sectional view for illustrating a step subsequent to the step illustrated in FIG. 7 of the administrable liquid medicine preparation method of the embodiment of the present invention;
FIG. 9 is a cross-sectional view for illustrating a step subsequent to the step illustrated in FIG. 8 of the administrable liquid medicine preparation method of the embodiment of the present invention;
FIG. 10 is a cross-sectional view for illustrating a step subsequent to the step illustrated in FIG. 9 of the administrable liquid medicine preparation method of the embodiment of the present invention;
FIG. 11 is a plan view of an administrable liquid medicine preparation tool according to another embodiment of the present invention;
FIG. 12 is a cross-sectional view taken along line B-B in FIG. 11;
FIG. 13 is a plan view of a housing member forming the administrable liquid medicine preparation tool illustrated in FIG. 11;
FIG. 14 is a plan view of an administrable liquid medicine preparation tool according to another embodiment of the present invention;
FIG. 15 is a cross-sectional view taken along line C-C in FIG. 14;
FIG. 16 is a plan view of a housing member forming the administrable liquid medicine preparation tool illustrated in FIG. 14;
FIG. 17 is a cross-sectional view of a variation of the administrable liquid medicine preparation tool illustrated in FIGS. 14 to 16;
FIG. 18 is a plan view of a housing member forming the administrable liquid medicine preparation tool illustrated in FIG. 17;
FIG. 19 is a cross-sectional view of another variation of the administrable liquid medicine preparation tool illustrated in FIGS. 14 to 16;
FIG. 20 is a plan view of an administrable liquid medicine preparation tool according to another embodiment of the present invention;
FIG. 21 is a cross-sectional view corresponding to FIG. 2 of the administrable liquid medicine preparation tool according to another embodiment of the present invention;
FIG. 22 is a cross-sectional view for illustrating a step of an administrable liquid medicine preparation method of another embodiment of the present invention using an administrable liquid medicine preparation kit including the administrable liquid medicine preparation tool illustrated in FIG. 21;
FIG. 23 is a cross-sectional view for illustrating a step subsequent to the step illustrated in FIG. 22 of the administrable liquid medicine preparation method of another embodiment of the present invention;
FIG. 24 is a cross-sectional view for illustrating a step subsequent to the step illustrated in FIG. 23 of the administrable liquid medicine preparation method of another embodiment of the present invention; and
FIG. 25 is a cross-sectional view for illustrating a step subsequent to the step illustrated in FIG. 24 of the administrable liquid medicine preparation method of another embodiment of the present invention.

### DETAILED DESCRIPTION

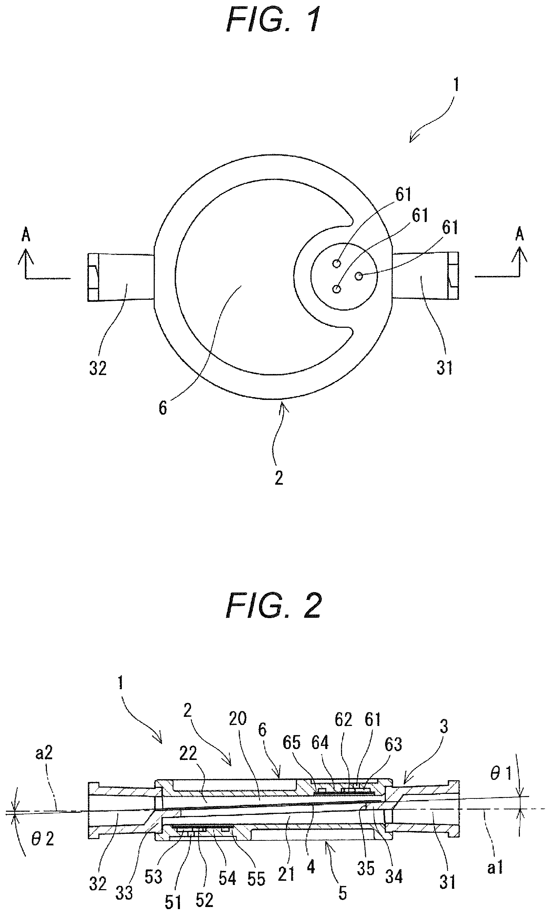

An administrable liquid medicine preparation tool of the present invention is described with reference to embodiments illustrated in the drawings. FIGS. 1 to 5 illustrate an administrable liquid medicine preparation tool 1 or some members forming the administrable liquid medicine preparation tool 1 in a predetermined state.

The administrable liquid medicine preparation tool 1 of the present invention is provided with a first port 31 to which a syringe is attachable, a second port 32 to which a syringe is attachable, a main body 2 provided with a liquid flow path 20 that communicates the first port 31 with the second port 32, a hydrophilic filtration membrane 4 that divides the liquid flow path 20 into a first port side flow path 21 and a second port side flow path 22, a first air discharge hole 51 that communicates the first port side flow path 21 with the outside, a first air-permeable membrane 52 that blocks the first air discharge hole 51 in an air-permeable manner, a second air discharge hole 61 that communicates the second port side flow path 22 with the outside, and a second air-permeable membrane 62 that blocks the second air discharge hole 61 in an air-permeable manner.

As illustrated in FIGS. 1 and 2, the administrable liquid medicine preparation tool 1 is provided with a housing member 3 with an upper surface and a lower surface (an upper side surface and a lower side surface in FIG. 2) opened, the housing member 3 including the first port 31 and the second port 32, a first lid member 5 fixed to one surface (herein, the lower surface) of the housing member 3, and a second lid member 6 fixed to the other surface (herein, the upper surface) of the housing member 3. In this embodiment, the housing member 3 and the two lid members 5 and 6 form the first port 31, the second port 32, and the main body 2.

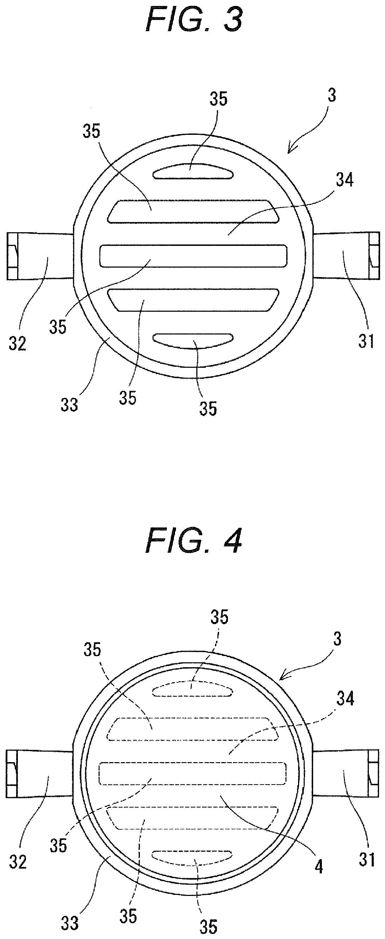

Specifically, as illustrated in FIGS. 2 and 3, the housing member 3 is provided with an annular side wall 33 formed into a short cylindrical shape, the first port 31 protruding outward from the side wall 33, and the second port 32 provided so as to face the first port 31 and protruding outward from the side wall 33. In the first port 31 and the second port 32, the inside and the outside of the housing member 3 (the side wall 33 in this embodiment) are communicated with each other.

In this embodiment, each of the first port 31 and the second port 32 forms a connection port including a female Luer taper compliant with an ISO standard (specifically, a female Luer taper compliant with ISO 594-1) to which a nozzle (including a male Luer taper compliant with ISO 594-1) of a normal syringe may be liquid-tightly connected.

The first port 31 and the second port 32 are preferably provided so as to face each other as is the case with the administrable liquid medicine preparation tool 1 of this embodiment. Especially, the first port 31 and the second port 32 are preferably provided so as to face each other with axes thereof substantially aligned with each other. As a result, usability such as operability is improved.

A partition plate 34 is provided inside the housing member 3 so as to separate the first port 31 from the second port 32. In a state illustrated in FIG. 2, the partition plate 34 is formed into an inclined shape with a height gradually increasing from the second port 32 side toward the first port 31 side. As illustrated in FIG. 3, a plurality of (herein, five) through holes 35 is formed on the partition plate 34. A predetermined liquid may flow between the first port side flow path 21 and the second port side flow path 22 in the housing member 3 via the through holes 35 and the hydrophilic filtration membrane 4 to be described later.

The hydrophilic filtration membrane (in other words, a liquid-permeable filter) 4 is arranged to be fixed on a surface on one side (herein, an upper side surface in FIG. 2) of the partition plate 34. The hydrophilic filtration membrane 4 is fixed to the partition plate 34 of the housing member 3 by heat sealing or adhesion. As illustrated in FIG. 4, the hydrophilic filtration membrane 4 has a size to close at least all of the plurality of openings 35 formed on the partition plate 34. The hydrophilic filtration membrane 4 divides the inside of the housing member 3 into the first port 31 side and the second port 32 side.

Note that, as the hydrophilic filtration membrane 4, a hydrophilic porous membrane, a hydrophilic nonwoven fabric and the like may be used. As a material of the hydrophilic filtration membrane, a hydrophilic material such as polysulfone, polyethersulfone, cellulose acetate, and nitrocellulose, and a hydrophobic membrane formed of polypropylene, polyethylene, polyester, polyvinylidene fluoride and the like subjected to hydrophilization treatment may also be used. As the hydrophilic filtration membrane 4 of this embodiment, one having poor air bubble permeability or substantially having air bubble impermeability is used when a liquid flows when a gasket of the syringe is pressed. In the hydrophilic filtration membrane 4, a bubble point is preferably 20 kPa or more, and especially preferably 50 kPa or more. Note that this bubble point is measured by a method specified in JIS K 3832 (ISO 4003).

In the hydrophilic filtration membrane 4, a pore diameter of a fine pore of the membrane is preferably 0.1 to 10 µm, and especially preferably 0.1 to 1.0 µm. A thickness of the membrane is preferably 10 to 300 µm, and especially preferably 20 to 200 µm.

In this embodiment, as illustrated in FIG. 2, the first port 31 is provided so that an axis a1 thereof intersects the hydrophilic filtration membrane 4 at an angle of 81, and further the second port 32 is provided so that an axis a2 thereof intersects the hydrophilic filtration membrane 4 at an angle of θ2. Note that each of the angles θ1 and θ2 is preferably 30° or smaller. Note that, in the administrable liquid medicine preparation tool 1 of this embodiment, the first port 31 and the second port 32 are provided so as to face each other with axes thereof substantially aligned with each other.

As illustrated in FIG. 2, a peripheral edge of the first lid member 5 forming the main body 2 of the administrable liquid medicine preparation tool 1 is liquid-tightly fixed to a peripheral edge of the housing member 3 on the lower surface of the housing member 3. In FIG. 2, an inner bottom surface of the first lid member 5 and a lower surface of the hydrophilic filtration membrane 4 are separated from each other by a predetermined length, and the first port side flow path 21 of the liquid flow path 20 is formed therebetween. The first lid member 5 is formed into a substantially disk shape corresponding to an outer shape of the side wall 33 of the housing member 3.

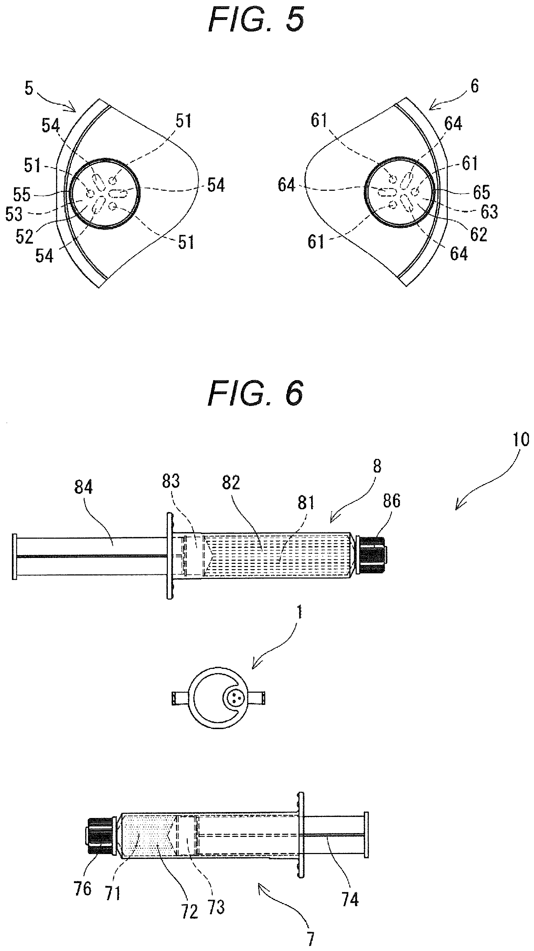

As illustrated in FIGS. 2 and 5, an air vent mechanism is formed on the first lid member 5. Specifically, the first lid member 5 is provided with the air vent mechanism in a position away from the first port 31 and close to the second port 32 in a state of being fixed to the housing member 3. In this embodiment, the air vent mechanism is provided with a plurality of (herein, three) first air discharge holes 51 and the first air-permeable membrane 52 provided so as to block them.

More specifically, as illustrated in FIGS. 2 and 5, the first lid member 5 is provided with a circular lower surface side recess on a lower surface thereof, and a circular recess 53 extending downward formed on an upper surface thereof in which three first air discharge holes 51 are provided. Note that the number of first air discharge holes is preferably about two to six. As illustrated in FIG. 5, the three first air discharge holes 51 are provided at substantially equal angular intervals around the center of the circular recess 53. In this embodiment, a plurality of (herein, three) air-permeable membrane supporting ribs 54 is provided inside the circular recess 53. The three air-permeable membrane supporting ribs 54 are provided between two adjacent first air discharge holes 51 at substantially equal angular intervals around the center of the circular recess 53.

As illustrated in FIGS. 2 and 5, an annular step 55 is formed at a peripheral edge of the circular recess 53 so as to surround the first air discharge holes 51. The first air-permeable membrane 52 is fixed on the annular step 55. The first air-permeable membrane 52 is a water-impermeable and thermoplastic air-permeable membrane, and is fixed on the annular step 55 by heat sealing or adhesion. The first air-permeable membrane 52 allows outflow of gas from the first air discharge holes 51 and prevents outflow of a liquid. Note that, as the first air-permeable membrane 52, a porous membrane formed of a thermoplastic resin or a nonwoven fabric is used. As a material for forming the first air-permeable membrane, a thermoplastic hydrophobic resin such as polypropylene, polyethylene, polysulfone, polyacrylonitrile, polytetrafluoroethylene, and cellulose acetate is used.

As illustrated in FIG. 2, a peripheral edge of the second lid member 6 forming the main body 2 of the administrable liquid medicine preparation tool 1 is liquid-tightly fixed to the peripheral edge of the housing member 3 on the upper surface of the housing member 3. In FIG. 2, a lower surface of the second lid member 6 and an upper surface of the hydrophilic filtration membrane 4 are away from each other by a predetermined length, and the second port side flow path 22 of the liquid flow path 20 is formed therebetween. As illustrated in FIG. 1, the second lid member 6 is formed into a substantially disk shape corresponding to the outer shape of the side wall 33 of the housing member 3.

As illustrated in FIG. 2, an air vent mechanism is formed on the second lid member 6. Specifically, the second lid member 6 is provided with the air vent mechanism in a position away from the second port 32 and close to the first port 31 in a state of being fixed to the housing member 3. In this embodiment, the air vent mechanism is provided with a plurality of (herein, three) second air discharge holes 61 and the second air-permeable membrane 62 provided so as to block them.

The second air-permeable membrane 62 allows outflow of gas from the second air discharge holes 61 and prevents outflow of a liquid. Note that, as the second air-permeable membrane 62, a porous membrane formed of a thermoplastic resin or a nonwoven fabric is used. As a material for forming the second air-permeable membrane, a thermoplastic hydrophobic resin such as polypropylene, polyethylene, polysulfone, polyacrylonitrile, polytetrafluoroethylene, and cellulose acetate is used.

Note that, in this embodiment, the second lid member 6 has substantially the same configuration as that of the first lid member 5 described above. That is, the second lid member 6 is provided with the three second air discharge holes 61, the second air-permeable membrane 62, a circular recess 63, three air-permeable membrane supporting ribs 64, and an annular step 65 as those forming the air vent mechanism; they correspond to the three first air discharge holes 51, the first air-permeable membrane 52, the circular recess 53, the three air-permeable membrane supporting ribs 54, and the annular step 55 in the first lid member 5, respectively, so that the detailed description thereof is omitted.

FIG. 6 illustrates an administrable liquid medicine preparation kit 10 of the present invention provided with the administrable liquid medicine preparation tool 1. The administrable liquid medicine preparation kit 10 of this embodiment is provided with the above-described administrable liquid medicine preparation tool 1, a first syringe 7 with stored a medicine 71 and connectable to the first port 31, and a second syringe 8 with stored a medical liquid 81 and connectable to the second port 32.

The first syringe 7 is provided with an outer cylinder 72, a gasket 73 slidably stored in the outer cylinder 72, and a plunger 74 for moving the gasket. Silicone oil may be applied to an inner surface of the outer cylinder 72 or an outer surface of the gasket 73. Since the silicon oil is applied, even in a case where air bubbles are likely to be generated, in the administrable liquid medicine preparation tool 1 and the administrable liquid medicine preparation kit 10 of the present invention, the air bubbles generated by a mixing operation (preparation step of mixed liquid) may be effectively removed. Slidability may also be imparted without using the silicone oil by providing a coating of a crosslinked product of a fluororesin or reactive silicone on the outer surface of the gasket in place of the silicone oil. In this manner, the generation of the air bubbles may be suppressed. A distal end (nozzle 75) of the outer cylinder 72 is sealed with a seal cap 76.

The first syringe 7 stores the medicine 71. The medicine 71 may be any medicine such as a liquid medicine diluted before administration to be used, in addition to a solidified injection such as a powdered medicine, a freeze-dried medicine of a medical product such as a low molecular compound or a biopharmaceutical (biological preparation). Examples of the medicine include, for example, antibiotic preparations such as antibiotics, antibacterial agents, and antifungal agents; chemotherapeutic agents such as antiviral agents and antimycotic agents; antitumor agents; central nervous system agents such as general anesthetics, antiepileptic agents, and psychoneurotic agents; peripheral nerve agents such as local anesthetics, skeletal muscle relaxants, and autonomic nerve agents; circulatory organ agents such as cardiotonic agents; digestive organ agents such as peptic ulcer agents; hormonal agents such as pituitary hormone agents, thyroid and parathyroid hormone agents, and adrenal hormone agents; narcotic preparations; blood preparations such as interferon preparations, human immunoglobulin preparations, and blood coagulation factor preparations; and biological preparations such as vaccines.

In a case where the powdered medicine is used as the medicine 71, the present invention is especially effective. Furthermore, in a case where a powdered biological preparation containing a protein and a surfactant as necessary and powdered by a freeze-drying method, a spray-drying method and the like is used as the powdered medicine, the present invention is especially effective. Note that, this may also be used for a liquid agent that requires a dissolution operation such as a remifentanil preparation.

Similarly to the first syringe 7, the second syringe 8 is also provided with an outer cylinder 82, a gasket 83 slidably stored in the outer cylinder 82, and a plunger 84 for moving the gasket. Silicone oil may be applied to an inner surface of the outer cylinder 82 or an outer surface of the gasket 83, and a coating made of a crosslinked product of a fluororesin or a reactive silicone may be provided in place of the silicone oil. A distal end (nozzle 85) of the outer cylinder 82 is sealed with a seal cap 86.

The second syringe 8 stores the medical liquid 81. As the medical liquid 81, a preparation preparing liquid (medicine solvent) such as distilled water for injection, physiological saline, or a glucose aqueous solution, and a liquid further containing a medicine (for example, vitamin and mineral) therein are used.

In this embodiment, the distal end (nozzle 75) of the first syringe 7 and the distal end (nozzle 85) of the second syringe 8 form male connection ports including male Luer tapers corresponding to the first port 31 and the second port 32 described above, respectively. As a result, as is described later, when the first syringe 7 is inserted into the first port 31 and the second syringe 8 is inserted into the second port 32, both of them may be connected air-tightly and liquid-tightly.

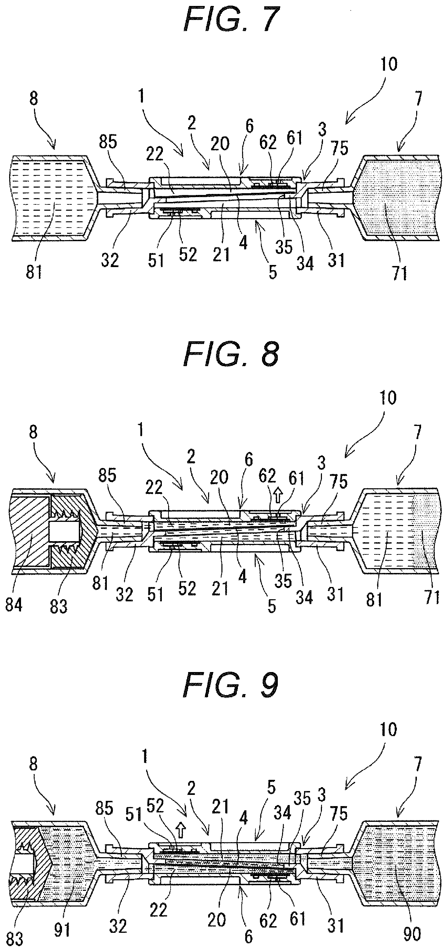

Subsequently, an administrable liquid medicine preparation method using the above-described administrable liquid medicine preparation kit 10 is described with reference to FIGS. 7 to 10.

The administrable liquid medicine preparation method using the above-described administrable liquid medicine preparation kit 10 includes a step of connecting the first syringe 7 to the first port 31 of the administrable liquid medicine preparation tool 1 and connecting the second syringe 8 to the second port 32 of the administrable liquid medicine preparation tool 1, a step of operating the second syringe 8 to cause the medical liquid 81 stored in the second syringe 8 to pass through the administrable liquid medicine preparation tool 1 and flow into the first syringe 7 after removing air bubbles therefrom via the second air discharge hole 61, and mixing the medicine 71 and the medical liquid 81 in the first syringe 7 to prepare a mixed liquid 90, and a step of operating the first syringe 7 to cause the mixed liquid 90 stored in the first syringe 7 to pass through the administrable liquid medicine preparation tool 1 and flow into the second syringe 8 after removing air bubbles therefrom via the first air discharge hole 51.

Specifically, first, as illustrated in FIG. 7, the seal cap 76 of the first syringe 7 is removed and the nozzle 75 thereof is inserted into the first port 31 of the administrable liquid medicine preparation tool 1 to be connected, and the seal cap 86 of the second syringe 8 is removed and the nozzle 85 thereof is inserted into the second port 32 of the administrable liquid medicine preparation tool 1 to be connected. In this embodiment, since the first port 31 and the second port 32 form female connection ports including female Luer tapers compliant with the ISO standard, and the nozzle 75 of the first syringe 7 and the nozzle 85 of the second syringe 8 form male connection ports including male Luer tapers corresponding to the first port 31 and the second port 32, respectively, they may be reliably connected air-tightly and liquid-tightly. At that time, the administrable liquid medicine preparation tool 1 is preferably arranged with the second lid member 6 on the upper side so that the second air discharge hole 61 opens upward. In this embodiment, the syringe may also be reversely attached; the first syringe 7 may be connected to the second port and the second syringe 8 may be connected to the first port.

Next, as illustrated in FIG. 8, the second syringe 8 is operated (the plunger 84 is pressed to advance the gasket 83) to cause the medical liquid 81 stored in the second syringe 8 to pass through the administrable liquid medicine preparation tool 1 and flow into the first syringe 7. At that time, in the liquid flow path 20 of the administrable liquid medicine preparation tool 1, air bubbles (foam) contained in the medical liquid 81 are prevented from flowing from the second port side flow path 22 to the first port side flow path 21 by the hydrophilic filtration membrane 4, they permeate the second air-permeable membrane 62 provided on the second lid member 6 forming the second port side flow path 22, and are released out of the administrable liquid medicine preparation tool 1 via the second air discharge hole 61 to be removed.

Note that, as described above, in this embodiment, the second port 32 is provided so that the axis a2 thereof forms the predetermined angle θ2 with respect to the hydrophilic filtration membrane 4, and the air vent mechanism provided with the plurality of second air discharge holes 61 and the second air-permeable membrane 62 is provided in a position away from the second port 32 and close to the first port 31. As a result, as the hydrophilic filtration membrane 4 is closer to the second air-permeable membrane 62, a distance therebetween becomes shorter, and the distance between the hydrophilic filtration membrane 4 and the second air-permeable membrane 62 in a portion where this faces the second air-permeable membrane 62 is the shortest. Therefore, the air bubbles may be more effectively released from the second air discharge holes 61.

The medical liquid 81 that passes through the hydrophilic filtration membrane 4 and the first port side flow path 21 flows into the first syringe 7. The medical liquid 81 that flows in is mixed with the medicine 71 in the first syringe 7, so that the mixed liquid 90 of the medicine 71 and the medical liquid 81 is prepared. At that time, since the air bubbles contained in the medical liquid 81 are removed in the administrable liquid medicine preparation tool 1, generation of air bubbles when the medicine 71 and the medical liquid 81 are mixed is suppressed.

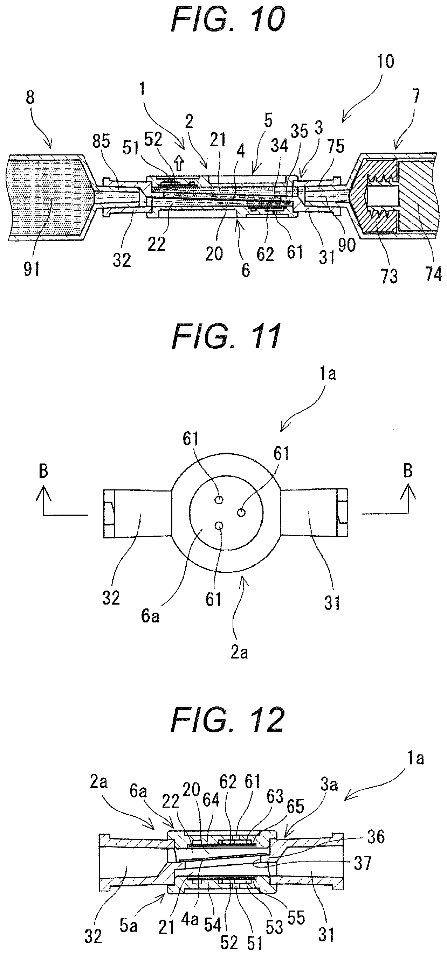

Then, as illustrated in FIGS. 9 and 10, the first syringe 7 is operated (the plunger 74 is pressed to advance the gasket 73) to cause the mixed liquid 90 stored in the first syringe 7 to pass through the administrable liquid medicine preparation tool 1 and flow into the second syringe 8. Note that, it is preferable to vertically reverse the administrable liquid medicine preparation tool 1 before performing this operation. That is, at that time, as illustrated in FIG. 9, the administrable liquid medicine preparation tool 1 is preferably arranged with the first lid member 5 on the upper side so that the first air discharge hole 51 opens upward.

In the liquid flow path 20 of the administrable liquid medicine preparation tool 1, air bubbles contained in the mixed liquid 90 are prevented from flowing from the first port side flow path 21 to the second port side flow path 22 by the hydrophilic filtration membrane 4, they permeate the first air-permeable membrane 52 provided on the first lid member 5 forming the first port side flow path 21, and are released out of the administrable liquid medicine preparation tool 1 via the first air discharge hole 51 to be removed. The mixed liquid 90 that passes through the hydrophilic filtration membrane 4 and the second port side flow path 22 from which the air bubbles are removed flows into the second syringe. As a result, it is possible to obtain the second syringe 8 storing an administrable liquid medicine 91 from which the air bubbles are removed that is administrable.

Note that, as described above, in this embodiment, the first port 31 is provided so that the axis a1 thereof forms the predetermined angle θ1 with respect to the hydrophilic filtration membrane 4, and the air vent mechanism provided with the plurality of first air discharge holes 51 and the first air-permeable membrane 52 is provided in a position away from the first port 31 and close to the second port 32. As a result, as the hydrophilic filtration membrane 4 is closer to the first air-permeable membrane 52, a distance therebetween becomes shorter, and the distance between the hydrophilic filtration membrane 4 and the first air-permeable membrane 52 in a portion where this faces the first air-permeable membrane 52 is the shortest. Therefore, the air bubbles may be more effectively released from the first air discharge holes 51.

In such administrable liquid medicine preparation tool 1, regardless of a flowing direction of the liquid such as the medical liquid 81 or the mixed liquid 90, air bubbles (foam) present in the liquid may be removed and generation of new air bubbles caused by such air bubbles may be suppressed by causing the liquid to pass through the administrable liquid medicine preparation tool 1. Furthermore, it is not necessary to exchange the first syringe 7 and the second syringe 8 when preparing the administrable liquid medicine 91 using the administrable liquid medicine preparation tool 1. Since the administrable liquid medicine 91 may be prepared by operating the syringes 7 and 8, a preparation operation of the administrable liquid medicine 91 may be more easily performed.

Note that the powdered biological preparation (biological preparation) containing the protein as an active ingredient (containing the protein and surfactant) has low solubility and tends to bubble. There also is a characteristic that air bubbles generated once are hard to disappear. The administrable liquid medicine preparation tool 1 of the present invention is especially effective in a case where such medicine is used.

The administrable liquid medicine preparation tool of the present invention may also be an administrable liquid medicine preparation tool 1a as illustrated in FIGS. 11 to 13. Note that, in the administrable liquid medicine preparation tool 1a, as for a configuration corresponding to that of the above-described administrable liquid medicine preparation tool 1, the same name and reference sign are used to omit the description, and as for some configurations, "a" is added at the end of the reference sign to omit the description other than a different point.

In the administrable liquid medicine preparation tool 1a of this embodiment, a substantially entire first lid member 5a and second lid member 6a form the air vent mechanism, and an outer shape of a main body 2a is made smaller than the outer shape of the main body 2 of the administrable liquid medicine preparation tool 1 described above. Therefore, the administrable liquid medicine preparation tool 1a may be made further smaller, and a volume of the administrable liquid medicine preparation tool 1a may be made smaller, so that a so-called dead volume may be made smaller. That is, an amount of the mixed liquid 90 (administrable liquid medicine 91) remaining in the administrable liquid medicine preparation tool 1a after the preparation of the administrable liquid medicine 91 may be made smaller.

Specifically, as illustrated in FIGS. 11 to 13, a housing member 3a is provided with an annular side wall 33a formed into a short cylindrical shape, the first port 31 protruding outward from the side wall 33a, and the second port 32 provided so as to face the first port 31 and protruding outward from the side wall 33a. In the first port 31 and the second port 32, the inside and the outside of the housing member 3a (the side wall 33a) are communicated with each other.

In this embodiment, each of the first port 31 and the second port 32 forms a connection port including a female Luer taper compliant with an ISO standard (specifically, a female Luer taper compliant with ISO 594-1) to which a nozzle (including a male Luer taper compliant with ISO 594-1) of a normal syringe may be liquid-tightly connected.

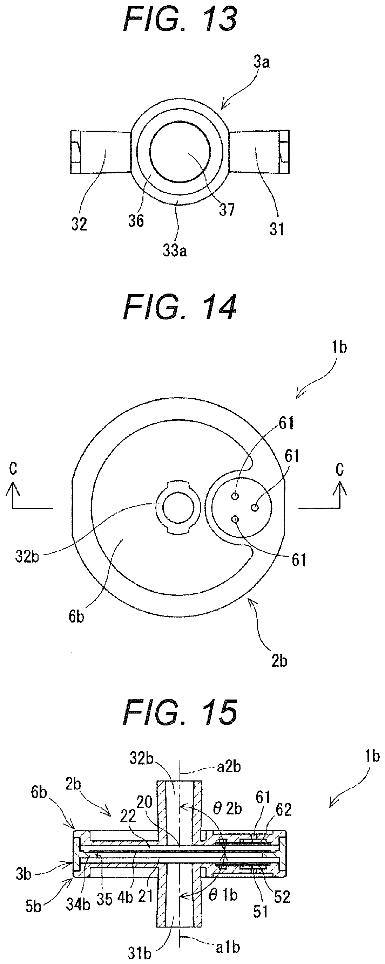

An annular inclined rib 36 is provided inside the housing member 3a so as to cross between the first port 31 and the second port 32. In a state illustrated in FIG. 12, the annular inclined rib 36 is formed into an inclined shape with a height gradually increasing from the second port 32 side toward the first port 31 side. As illustrated in FIGS. 12 and 13, a disk-shaped inclined opening 37 is formed in the annular inclined rib 36. A predetermined liquid may flow between the first port side flow path 21 and the second port side flow path 22 in the housing member 3a via the inclined opening 37 and a hydrophilic filtration membrane 4a.

The hydrophilic filtration membrane (liquid-permeable filter) 4a is arranged to be fixed on a surface on one side (herein, an upper side surface in FIG. 12) of the inclined opening 37. A peripheral edge of the hydrophilic filtration membrane 4a is fixed to the annular inclined rib 36 by heat sealing or adhesion. As illustrated in FIG. 12, the hydrophilic filtration membrane 4a has a size to close an entire inclined opening 37. The hydrophilic filtration membrane 4a divides the inside of the housing member 3a into a first port 31 side and a second port 32 side, in other words, the first port side flow path 21 and the second port side flow path 22.

The administrable liquid medicine preparation tool of the present invention may also be an administrable liquid medicine preparation tool 1b as illustrated in FIGS. 14 to 16. Note that, in the administrable liquid medicine preparation tool 1b, as for a configuration corresponding to that of the above-described administrable liquid medicine preparation tool 1, the same name and reference sign are used to omit the description, and as for some configurations, "b" is added at the end of the reference sign to omit the description other than a different point.

In the administrable liquid medicine preparation tool 1b, as illustrated in FIG. 15, a first port 31b is provided so as to protrude from a substantially disk-shaped first lid member 5b, and a second port 32b is provided so as to protrude from a substantially disk-shaped second lid member 6b. In this embodiment, the first port 31b and the second port 32b are arranged so as to face each other, and an axis of the first port 31b and an axis of the second port 32b intersect the hydrophilic filtration membrane 4b at an angle of 70° to 90°.

Specifically, the first port 31b is provided so that an axis a1b thereof intersects (is substantially orthogonal to) the hydrophilic filtration membrane 4b at an angle 81b of substantially 90°, and further the second port 32b is provided so that an axis a2b thereof intersects (is substantially orthogonal to) the hydrophilic filtration membrane 4b at an angle 02b of substantially 90°.

Note that the first port 31b and second port 32b may be slightly inclined with respect to the hydrophilic filtration membrane 4b. Specifically, the first port 31b may be provided so that the axis a1b thereof intersects the hydrophilic filtration membrane 4b at an angle of 70° to 90°, and further the second port 32b may be provided so that the axis a2b thereof intersects the hydrophilic filtration membrane 4b at an angle of 70° to 90° or smaller.

The housing member 3b is not provided with the first port or the second port. A partition plate 34b is provided inside the housing member 3b (main body 2b) so as to separate the first port 31b from the second port 32b. As illustrated in FIG. 15, the partition plate 34b is formed substantially horizontally at substantially the center of the housing member 3b.

In such administrable liquid medicine preparation tool 1b, a dimension in a vertical direction (the dimension and thickness in the vertical direction in FIG. 15) of the housing member 3b may be made smaller than that of the administrable liquid medicine preparation tool 1 described above. Therefore, a volume (so-called dead volume) of the administrable liquid medicine preparation tool 1b may be made smaller.

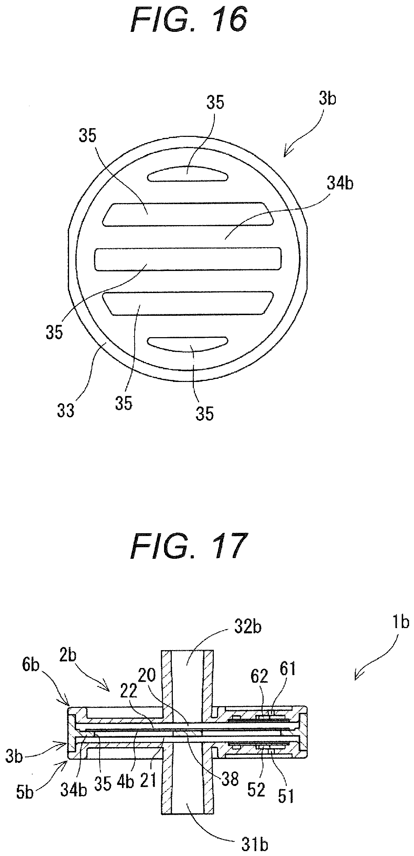

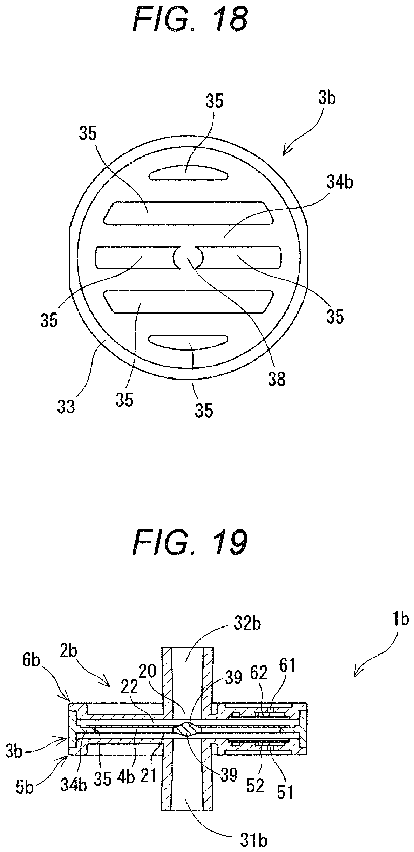

Note that, in the administrable liquid medicine preparation tool 1b, a flow of a liquid flowing from each of the ports 31b and 32b into the administrable liquid medicine preparation tool 1b is directly directed to the hydrophilic filtration membrane 4b. Therefore, for example, as illustrated in FIGS. 17 and 18, it is possible that the partition plate 34b is not provided with the through hole 35 in a portion immediately below (above) each of the ports 31b and 32b (in other words, this is provided with a membrane support 38). Furthermore, as illustrated in FIG. 19, a raised portion 39 may be provided in a portion immediately below (above) each of the ports 31b and 32b of the partition plate 34b to disperse the flow of the liquid.

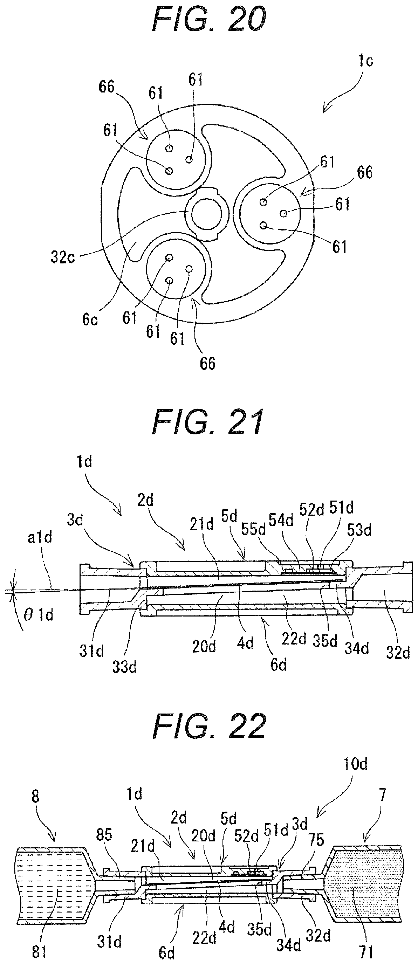

The administrable liquid medicine preparation tool of the present invention may also be an administrable liquid medicine preparation tool 1c as illustrated in FIG. 20. Note that the administrable liquid medicine preparation tool 1c has substantially the same configuration as that of the administrable liquid medicine preparation tool 1b described above, and is different therefrom in that three air vent mechanisms 66 provided with the three second air discharge holes 61, the second air-permeable membrane 62, the circular recess 63, the three air-permeable membrane supporting ribs 64, and the annular step 65 are provided so as to surround the second port 32c on the second lid member 6c as illustrated in FIG. 20. Although not illustrated, the three air vent mechanisms are similarly provided on the first lid member 5c. As a result, an air bubble removing function in the administrable liquid medicine preparation tool 1c is enhanced. For other configurations, the configuration described in the administrable liquid medicine preparation tool 1b may be used.

The administrable liquid medicine preparation tool of the present invention may also be an administrable liquid medicine preparation tool 1d as illustrated in FIG. 21. Note that, in the administrable liquid medicine preparation tool 1d, as for a configuration corresponding to that of the above-described administrable liquid medicine preparation tool 1, the same name is used to omit the detailed description, and "d" is added at the end of all the reference signs.

The administrable liquid medicine preparation tool 1d is provided with a first port 31d to which a syringe is attachable, a second port 32d to which a syringe is attachable, a main body 2d provided with a liquid flow path 20d that communicates the first port 31d with the second port 32d, a hydrophilic filtration membrane 4d having poor air bubble permeability that divides the liquid flow path 20d into a first port side flow path 21d and a second port side flow path 22d, a first air discharge hole 51d that communicates the first port side flow path 21d with the outside, and a first air-permeable membrane 52d that blocks the first air discharge hole 51d in an air-permeable manner, in which each of the first port 31d and the second port 32d forms a female connection port including a female Luer taper compliant with the ISO standard.

As illustrated in FIG. 21, in the administrable liquid medicine preparation tool 1d of this embodiment, the first port 31d and the second port 32d are arranged so as to face each other, and an axis aid of the first port 31d intersects the hydrophilic filtration membrane 4d at a predetermined angle θ1d, and further the first air discharge hole 51d is provided in a position away from the first port 31d and is close to the second port 32d. The angle θ1d is preferably 30° or smaller.

As illustrated in FIG. 21, a peripheral edge of a first lid member 5d forming the main body 2d of the administrable liquid medicine preparation tool 1d is liquid-tightly fixed to a peripheral edge of a housing member 3d on an upper surface of the housing member 3d. The first lid member 5d is provided with an air vent mechanism in a position away from the first port 31d and close to the second port 32d in a state of being fixed to the housing member 3d. In this embodiment, the air vent mechanism is provided with a plurality of (herein, three) first air discharge holes 51d and the first air-permeable membrane 52d provided so as to block them. In contrast, a second lid member 6d that forms the main body 2d of the administrable liquid medicine preparation tool 1d with a peripheral edge liquid-tightly fixed to the peripheral edge of the housing member 3d on a lower surface of the housing member 3d is not provided with such air vent mechanism.

The administrable liquid medicine preparation tool 1d of this embodiment forms an administrable liquid medicine preparation kit 10d provided with the first syringe 7 with stored the medicine 71 and connectable to the first port 31d and the second port 32d, and the second syringe 8 with stored the medical liquid 81 and connectable to the first port 31d and the second port 32d.

Then, as illustrated in FIGS. 22 to 25, the administrable liquid medicine preparation method using such administrable liquid medicine preparation kit 10d includes a step of connecting the second syringe 8 to the first port 31d and connecting the first syringe 7 to the second port 32d, a step of operating the second syringe 8 to cause the medical liquid 81 stored in the second syringe 8 to pass through the administrable liquid medicine preparation tool 1d and flow into the first syringe 7 after removing air bubbles therefrom via the first air discharge hole 51d, and mixing the medicine 71 and the medical liquid 81 in the first syringe 7 to prepare the mixed liquid 90, a syringe attaching port change step of separating the first syringe 7 and the second syringe 8 from the administrable liquid medicine preparation tool 1d, connecting the first syringe 7 storing the mixed liquid 90 to the first port 31d, and connecting the second syringe 8 to the second port 32d, and a step of operating the first syringe 7 to cause the mixed liquid 90 stored in the first syringe 7 to pass through the administrable liquid medicine preparation tool 1d and flow into the second syringe 8 after removing air bubbles therefrom via the first air discharge hole 51d.

Specifically, first, as illustrated in FIG. 22, the nozzle 85 of the second syringe 8 is inserted into and connected to the first port 31d, and the nozzle 75 of the first syringe 7 is inserted into and connected to the second port 32d. At that time, the administrable liquid medicine preparation tool 1d is preferably arranged with the first lid member 5d on the upper side so that the first air discharge hole 51d opens upward.

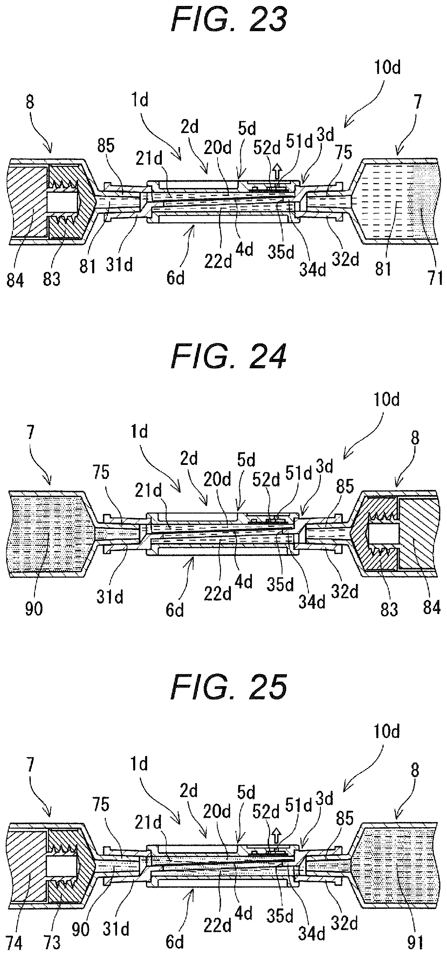

Next, as illustrated in FIG. 23, the second syringe 8 is operated (the plunger 84 is pressed to advance the gasket 83) to cause the medical liquid 81 stored in the second syringe 8 to pass through the administrable liquid medicine preparation tool 1d and flow into the first syringe 7. At that time, in the liquid flow path 20d of the administrable liquid medicine preparation tool 1d, air bubbles contained in the medical liquid 81 are prevented from flowing from the first port side flow path 21d to the second port side flow path 22d by the hydrophilic filtration membrane 4d, they permeate the first air-permeable membrane 52d provided on the first lid member 5d forming the first port side flow path 21d, and are released out of the administrable liquid medicine preparation tool 1d via the first air discharge hole 51d to be removed.

Note that, as described above, in this embodiment, the first port 31d is provided so that the axis a1d thereof forms the predetermined angle 81d with respect to the hydrophilic filtration membrane 4d, and the air vent mechanism provided with the plurality of first air discharge holes 51d and the first air-permeable membrane 52d is provided in a position away from the first port 31d and close to the second port 32d. As a result, as the hydrophilic filtration membrane 4d is closer to the first air-permeable membrane 52d, a distance therebetween becomes shorter, and the distance between the hydrophilic filtration membrane 4d and the first air-permeable membrane 52d in a portion where this faces the first air-permeable membrane 52d is the shortest. Therefore, the air bubbles may be more effectively released from the first air discharge holes 51d.

The medical liquid 81 that passes through the hydrophilic filtration membrane 4d and the first port 31d flows into the first syringe 7. The medical liquid 81 that flows in is mixed with the medicine 71 in the first syringe 7, so that the mixed liquid 90 of the medicine 71 and the medical liquid 81 is prepared. At that time, since the air bubbles contained in the medical liquid 81 are removed in the administrable liquid medicine preparation tool 1d, generation of air bubbles when the medicine 71 and the medical liquid 81 are mixed is suppressed.

Thereafter, the first syringe 7 and the second syringe 8 are separated from the administrable liquid medicine preparation tool 1d, the first syringe 7 storing the mixed liquid 90 is connected to the first port 31d, and the second syringe 8 is connected to the second port 32d. That is, the first syringe 7 and the second syringe 8 are exchanged. At that time, the administrable liquid medicine preparation tool 1d is preferably arranged with the first lid member 5 on the upper side so that the first air discharge hole 51d opens upward.

Then, as illustrated in FIGS. 24 and 25, the first syringe 7 is operated (the plunger 74 is pressed to advance the gasket 73) to cause the mixed liquid 90 stored in the first syringe 7 to pass through the administrable liquid medicine preparation tool 1d and flow into the second syringe 8. At that time, in the liquid flow path 20d of the administrable liquid medicine preparation tool 1d, air bubbles contained in the mixed liquid 90 are prevented from flowing from the first port side flow path 21d to the second port side flow path 22d by the hydrophilic filtration membrane 4d, they permeate the first air-permeable membrane 52d provided on the first lid member 5d forming the first port side flow path 21d, and are released out of the administrable liquid medicine preparation tool 1d via the first air discharge hole 51d to be removed. The mixed liquid 90 that passes through the hydrophilic filtration membrane 4d and the second port 32d from which the air bubbles are removed flows into the second syringe 8. As a result, it is possible to obtain the second syringe 8 storing an administrable liquid medicine 91 from which the air bubbles are removed that is administrable.

Herein also, as described above, in this embodiment, since the distance between the hydrophilic filtration membrane 4d and the first air-permeable membrane 52d in a portion facing the first air-permeable membrane 52d is short, the air bubbles may be more effectively released from the first air discharge hole 51d.

By using such administrable liquid medicine preparation tool 1d, the air bubbles (foam) present in the liquid such as the medical liquid 81 or the mixed liquid 90 may be removed by causing them to pass through the administrable liquid medicine preparation tool 1d, and generation of new air bubbles caused by the presence of such air bubbles may be suppressed. Furthermore, in the administrable liquid medicine preparation tool 1d, since both the first syringe 7 and the second syringe 8 may be connected to both the first port 31d and the second port 32d, it is possible to cause the liquid to pass through the administrable liquid medicine preparation tool 1d by appropriately exchanging the syringes 7 and 8 to operate, so that the preparation operation of the administrable liquid medicine becomes relatively easy.

The administrable liquid medicine preparation tool of the present invention is provided with a first port to which a syringe is attachable, a second port to which a syringe is attachable, a main body provided with a liquid flow path that communicates the first port with the second port, a hydrophilic filtration membrane that divides the liquid flow path into a first port side flow path and a second port side flow path, a first air discharge hole that communicates the first port side flow path with the outside, a first air-permeable membrane that blocks the first air discharge hole in an air-permeable manner, a second air discharge hole that communicates the second port side flow path with the outside, and a second air-permeable membrane that blocks the second air discharge hole in an air-permeable manner. Therefore, by causing the liquid to pass through the administrable liquid medicine preparation tool, air bubbles (foam) present in the liquid may be removed, and air bubbles in the administrable liquid medicine to be prepared may be reduced. Furthermore, since it is possible to cause the liquid to pass through the administrable liquid medicine preparation tool by operating the syringe, a preparation operation of the administrable liquid medicine may be more easily performed.

## Claims

1. An administrable liquid medicine preparation tool comprising:
a first port (31) ;
a second port (32) ;
a main body (2, 2a, 2b, 2d) provided with a liquid flow path (20) that communicates the first port (31) with the second port (32) ;
a hydrophilic filtration membrane (4) that divides the liquid flow path (20) into a first port side flow path (21) and a second port side flow path (22);
**characterized in that**
the administrable liquid medicine preparation tool is an administrable mixed liquid medicine preparation tool,
the administrable mixed liquid medicine preparation tool comprises a first air discharge hole (51, 51d) that communicates the first port side flow path (21) with an outside; a first air-permeable membrane (52, 52d) that blocks the first air discharge hole (51, 51d) in an air-permeable manner; a second air discharge hole (61) that communicates the second port side flow path (22) with the outside; and a second air-permeable membrane (62) that blocks the second air discharge hole (61) in an air-permeable manner
the first port (31) is a female connection port including a female Luer taper compliant with an ISO standard and capable of attaching a first syringe (7),
the second port (32) is a female connection port including a female Luer taper compliant with an ISO standard and capable of attaching a second syringe (8), and
the hydrophilic filtration membrane (4) has air bubble impermeability.

2. The administrable liquid medicine preparation tool according to claim 1 ,
wherein
the first port (31) is provided so that an axial direction of the first port (31) intersects the hydrophilic filtration membrane (4), and further the second port (32) is provided so that an axial direction of the second port (32) intersects the hydrophilic filtration membrane (4).

3. The administrable liquid medicine preparation tool according to claim 2,
wherein
the first port (31) and the second port (32) are arranged so as to face each other, and an axis of the first port (31) and an axis of the second port (32) intersect the hydrophilic filtration membrane (4) at an angle of 30° or smaller.

4. The administrable liquid medicine preparation tool according to claim 2,
wherein
the first port (31) and the second port (32) are arranged so as to face each other, and an axis of the first port (31) and an axis of the second port (32) intersect the hydrophilic filtration membrane (4) at an angle of 70° to 90°.

5. The administrable liquid medicine preparation tool according to any one of claims 1 to 4, wherein
the hydrophilic filtration membrane (4) has a pore diameter of a fine pore of the membrane of 0.1 to 10 µm, and especially preferably 0.1 to 1.0 µm and a thickness of the membrane is 10 to 300 µm, and especially preferably 20 to 200 µm.

6. An administrable liquid medicine preparation kit comprising:
the administrable liquid medicine preparation tool according to any one of claims 1 to 5; wherein
the first syringe (7) has a medicine (71) stored therein and is connectable to the first port (31); and
the second syringe (8) has medical liquid (81) stored therein and is connectable to the second port (32).

7. The administrable liquid medicine preparation kit according to claim 6,
wherein
the second syringe (8) with stored a powdered preparation preparing liquid.

8. An administrable liquid medicine preparation method using the administrable liquid medicine preparation kit according to claim 6, comprising :
a step of connecting the first syringe (7) to the first port (31) and connecting the second syringe (8) to the second port (32);
a step of operating the second syringe (8) to cause the medical liquid (81) stored in the second syringe (8) to pass through the administrable liquid medicine preparation tool and flow into the first syringe (7) after removing air bubbles via the second air discharge hole (61), and mixing the medicine (71) and the medical liquid (81) in the first syringe (7) to prepare a mixed liquid (90); and
a step of operating the first syringe (7) to cause the mixed liquid (90) stored in the first syringe (7) to pass through the administrable liquid medicine preparation tool and flow into the second syringe (8) after removing air bubbles via the first air discharge hole (51, 51d).

## Patentansprüche

1. Werkzeug zur Zubereitung von verabreichbaren flüssigen Medikamenten, umfassend:
einen ersten Anschluss (31);
einen zweiten Anschluss (32);
einen Hauptkörper (2, 2a, 2b, 2d), der mit einem Flüssigkeitsströmungsweg (20) versehen ist, der den ersten Anschluss (31) mit dem zweiten Anschluss (32) verbindet;
eine hydrophile Filtrationsmembran (4), die den Flüssigkeitsströmungsweg (20) in einen ersten anschlussseitigen Strömungsweg (21) und einen zweiten anschlussseitigen Strömungsweg (22) unterteilt;
**dadurch gekennzeichnet, dass**
das Werkzeug zur Zubereitung von verabreichbaren flüssigen Medikamenten ein Werkzeug zur Zubereitung von gemischten, verabreichbaren flüssigen Medikamenten ist,
das Werkzeug zur Zubereitung von gemischten, verabreichbaren flüssigen Medikamenten ein erstes Luftauslassloch (51, 51d), das den ersten anschlussseitigen Strömungsweg (21) mit einer Außenseite verbindet; eine erste luftdurchlässige Membran (52, 52d), die das erste Luftauslassloch (51, 51d) in einer luftdurchlässigen Weise blockiert; ein zweites Luftauslassloch (61), das den zweiten anschlussseitigen Strömungsweg (22) mit der Außenseite verbindet; und eine zweite luftdurchlässige Membran (62), die das zweite Luftauslassloch (61) in einer luftdurchlässigen Weise blockiert, umfasst,
der erste Anschluss (31) ein weiblicher Verbindungsanschluss ist, der einen weiblichen Luer-Konus gemäß einer ISO-Norm aufweist und in der Lage ist, eine erste Spritze (7) daran anzubringen,
der zweite Anschluss (32) ein weiblicher Verbindungsanschluss ist, der einen weiblichen Luer-Konus gemäß einer ISO-Norm aufweist und in der Lage ist, eine zweite Spritze (8) daran anzubringen, und
die hydrophile Filtrationsmembran (4) eine Undurchlässigkeit für Luftblasen aufweist.

2. Werkzeug zur Zubereitung von verabreichbaren flüssigen Medikamenten nach Anspruch 1, wobei
der erste Anschluss (31) so vorgesehen ist, dass eine axiale Richtung des ersten Anschlusses (31) die hydrophile Filtrationsmembran (4) schneidet, und ferner der zweite Anschluss (32) so vorgesehen ist, dass eine axiale Richtung des zweiten Anschlusses (32) die hydrophile Filtrationsmembran (4) schneidet.

3. Werkzeug zur Zubereitung von verabreichbaren flüssigen Medikamenten nach Anspruch 2, wobei
der erste Anschluss (31) und der zweite Anschluss (32) so angeordnet sind, dass sie einander gegenüberliegen, und eine Achse des ersten Anschlusses (31) und eine Achse des zweiten Anschlusses (32) die hydrophile Filtrationsmembran (4) in einem Winkel von 30° oder weniger schneiden.

4. Werkzeug zur Zubereitung von verabreichbaren flüssigen Medikamenten nach Anspruch 2, wobei
der erste Anschluss (31) und der zweite Anschluss (32) so angeordnet sind, dass sie einander gegenüberliegen, und eine Achse des ersten Anschlusses (31) und eine Achse des zweiten Anschlusses (32) die hydrophile Filtrationsmembran (4) in einem Winkel von 70° bis 90° schneiden.

5. Werkzeug zur Zubereitung von verabreichbaren flüssigen Medikamenten nach einem der Ansprüche 1 bis 4, wobei
die hydrophile Filtrationsmembran (4) einen Porendurchmesser einer feinen Pore der Membran von 0,1 bis 10 µm, besonders bevorzugt 0,1 bis 1,0 µm, aufweist und eine Dicke der Membran 10 bis 300 µm, besonders bevorzugt 20 bis 200 um, beträgt.

6. Kit zur Zubereitung von verabreichbaren flüssigen Medikamenten, umfassend:
das Werkzeug zur Zubereitung von verabreichbaren flüssigen Medikamenten nach einem der Ansprüche 1 bis 5; wobei
die erste Spritze (7) ein Medikament (71) darin gespeichert aufweist und mit dem ersten Anschluss (31) verbunden werden kann; und
die zweite Spritze (8) eine medizinische Flüssigkeit (81) darin gespeichert aufweist und mit dem zweiten Anschluss (32) verbunden werden kann.

7. Kit zur Zubereitung von verabreichbaren flüssigen Medikamenten nach Anspruch 6, wobei
die zweite Spritze (8) eine pulverförmige Zubereitung zur Herstellung einer Flüssigkeit enthält.

8. Verfahren zur Zubereitung von verabreichbaren flüssigen Medikamenten unter Verwendung des Kits zur Zubereitung von verabreichbaren flüssigen Medikamenten nach Anspruch 6, umfassend:
einen Schritt des Verbindens der ersten Spritze (7) mit dem ersten Anschluss (31) und des Verbindens der zweiten Spritze (8) mit dem zweiten Anschluss (32);
einen Schritt des Betätigens der zweiten Spritze (8), um zu bewirken, dass die medizinische Flüssigkeit (81), die in der zweiten Spritze (8) gespeichert ist, durch das Werkzeug zur Zubereitung von verabreichbaren flüssigen Medikamenten hindurchgeht und in die erste Spritze (7) fließt, nachdem Luftblasen über das zweite Luftauslassloch (61) entfernt wurden, und das Medikament (71) und die medizinische Flüssigkeit (81) in der ersten Spritze (7) gemischt werden, um eine gemischte Flüssigkeit (90) herzustellen; und
einen Schritt des Betätigens der ersten Spritze (7), um zu bewirken, dass die gemischte Flüssigkeit (90), die in der ersten Spritze (7) gespeichert ist, durch das Werkzeug zur Zubereitung von verabreichbaren flüssigen Medikamenten hindurchgeht und in die zweite Spritze (8) fließt, nachdem Luftblasen durch das erste Luftauslassloch (51, 51d) entfernt wurden.

## Revendications

1. Outil de préparation médicale liquide administrable comprenant :
un premier orifice (31) ;
un deuxième orifice (32) ;
un corps principal (2, 2a, 2b, 2d) doté d'une voie d'écoulement de liquide (20) qui fait communiquer le premier orifice (31) avec le deuxième orifice (32) ;
une membrane de filtration hydrophile (4) qui divise la voie d'écoulement de liquide (20) en une voie d'écoulement latéral de premier orifice (21) et une voie d'écoulement latéral de deuxième orifice (22) ;
**caractérisé en ce que**
l'outil de préparation médicale liquide administrable est un outil de préparation médicale liquide mélangée administrable,
l'outil de préparation médicale liquide mélangée administrable comprend un premier trou d'évacuation d'air (51, 51d) qui fait communiquer la voie d'écoulement latéral de premier orifice (21) avec un extérieur ; une première membrane perméable à l'air (52, 52d) qui bouche le premier trou d'évacuation d'air (51, 51d) d'une manière perméable à l'air ; un deuxième trou d'évacuation d'air (61) qui fait communiquer la voie d'écoulement latéral de deuxième orifice (22) avec l'extérieur ; et une deuxième membrane perméable à l'air (62) qui bouche le deuxième trou d'évacuation d'air (61) d'une manière perméable à l'air le premier orifice (31) est un orifice de raccordement femelle comportant un raccord conique Luer femelle conforme à une norme ISO et apte à fixer une première seringue (7),
le deuxième orifice (32) est un orifice de raccordement femelle comportant un raccord conique Luer femelle conforme à une norme ISO et apte à fixer une deuxième seringue (8), et
la membrane de filtration hydrophile (4) présente une imperméabilité aux bulles d'air.

2. Outil de préparation médicale liquide administrable selon la revendication 1,
dans lequel
le premier orifice (31) est prévu de sorte qu'une direction axiale du premier orifice (31) croise la membrane de filtration hydrophile (4), et en outre le deuxième orifice (32) est prévu de sorte qu'une direction axiale du deuxième orifice (32) croise la membrane de filtration hydrophile (4) .

3. Outil de préparation médicale liquide administrable selon la revendication 2,
dans lequel
le premier orifice (31) et le deuxième orifice (32) sont agencés de manière à se faire face, et un axe du premier orifice (31) et un axe du deuxième orifice (32) croisent la membrane de filtration hydrophile (4) à un angle inférieur ou égal à 30°.

4. Outil de préparation médicale liquide administrable selon la revendication 2,
dans lequel
le premier orifice (31) et le deuxième orifice (32) sont agencés de manière à se faire face,
et un axe du premier orifice (31) et un axe du deuxième orifice (32) croisent la membrane de filtration hydrophile (4) à un angle de 70° à 90°.

5. Outil de préparation médicale liquide administrable selon l'une quelconque des revendications 1 à 4, dans lequel
la membrane de filtration hydrophile (4) présente un diamètre de pore d'un pore fin de la membrane de 0,1 à 10 um, et de manière particulièrement préférée 0,1 à 1,0 um et une épaisseur de la membrane est 10 à 300 µm, et de manière particulièrement préférée 20 à 200 µm.

6. Kit de préparation médicale liquide administrable comprenant :
l'outil de préparation médicale liquide administrable selon l'une quelconque des revendications 1 à 5 ; dans lequel
la première seringue (7) présente un médicament (71) stocké dans celle-ci et peut être raccordée au premier orifice (31) ; et
la deuxième seringue (8) présente un liquide médical (81) stocké dans celle-ci et peut être raccordée au deuxième orifice (32).

7. Kit de préparation médicale liquide administrable selon la revendication 6,
dans lequel
la deuxième seringue (8) stocke un liquide de préparation d'une préparation en poudre.

8. Procédé de préparation médicale liquide administrable au moyen du kit de préparation médicale liquide administrable selon la revendication 6, comprenant :
une étape de raccordement de la première seringue (7) au premier orifice (31) et de raccordement de la deuxième seringue (8) au deuxième orifice (32) ;
une étape d'actionnement de la deuxième seringue (8) pour amener le liquide médical (81) stocké dans la deuxième seringue (8) à passer à travers l'outil de préparation médicale liquide administrable et s'écouler dans la première seringue (7) après élimination des bulles d'air par le biais du deuxième trou d'évacuation d'air (61), et mélange du médicament (71) et du liquide médical (81) dans la première seringue (7) pour préparer un liquide mélangé (90) ; et
une étape d'actionnement de la première seringue (7) pour amener le liquide mélangé (90) stocké dans la première seringue (7) à passer à travers l'outil de préparation médicale liquide administrable et s'écouler dans la deuxième seringue (8) après élimination des bulles d'air par le biais du premier trou d'évacuation d'air (51, 51d).
